# EUROPÄISCHE PATENTSCHRIFT

(11) **EP 3 468 628 B1**
(45) Veröffentlichungstag und Bekanntmachung des Hinweises auf die Patenterteilung: **28.04.2021**
(21) Anmeldenummer: 17731823.5
(22) Anmeldetag: 02.06.2017
(51) Int. Cl.: A61L 27/10, A61F 2/28, A61B 17/80, A61C 8/00, C04B 35/119, C04B 35/493

(54) **OSTEOSYNTHESEKOERPER AUS ZIRKONDIOXIDKERAMIK**
OSTEOSYNTHESIS BODY CONSISTING OF ZIRCONIUM DIOXIDE CERAMIC
CORPS D'OSTÉOSYNTHÈSE EN CÉRAMIQUE ZIRCONE

(30) Priorität: 09.06.2016 DE 102016110512
(43) Veröffentlichungstag der Anmeldung: 17.04.2019
(73) Patentinhaber: Gahlert, Michael, 81925 München (DE); Gahlert, Stefan, 70619 Stuttgart (DE)
(72) Erfinder: Gahlert, Michael, 81925 München (DE); Gahlert, Stefan, 70619 Stuttgart (DE)
(74) Vertreter: Witte, Weller & Partner Patentanwälte mbB
(86) Internationale Anmeldenummer: PCT/EP2017/063548
(87) Internationale Veröffentlichungsnummer: WO 2017/211739

(56) Entgegenhaltungen:
- WO-A1-2015/181057

## Beschreibung

Die Erfindung betrifft Osteosynthesekörper. In der Unfallchirurgie werden seit den sechziger Jahren für die offene Reposition und die interne Fixation von Knochenfrakturen Osteosynthesekörper aus Metall verwendet. Anfangs wurde hierzu praktisch ausschließlich rostfreier Edelstahl verwendet. Allerdings zeigte sich, dass Osteosynthesestahl nicht völlig frei von Korrosion ist und zur Auslösung von Metallunverträglichkeiten und Allergien führen kann. Als problematisch haben sich insbesondere die teilweise toxischen Effekte von Chrom, Nickel und Eisen herausgestellt. Seit Jahrzehnten werden daher für die Osteosynthese Körper aus Titan verwendet, die wegen ihrer Oberflächenpassivierung aus Titandioxid als weitgehend bioinert angesehen werden.

Zwar führt eine Verwendung von Titan zu deutlich geringeren Infektionsraten und allergischen Reaktionen, jedoch treten vereinzelt dennoch Unverträglichkeitsreaktionen auf.

Ferner ist durch die CN 2862995 Y ein interner Fixierer aus einem Verbundmaterial aus Hydroxyapatit und Zirkondioxid bekannt. Allerdings handelt es sich hierbei um ein Verbundmaterial, also einer Kombination von Hydroxyapatit und Zirkondioxid. Der nähere Aufbau und die technische Ausführung dieses Verbundmaterials sind dieser Schrift nicht entnehmbar.

Aus der WO 2015/181057 A1 ist ein Osteosyntesevorrichtung für Knochenfrakturen mit einem Spannelement bekannt, das mittels Metallschrauben zu befestigen ist. Das Spannelement kann aus einem Metall, wie etwa einem Chrom-Nickel-Stahl, oder aus einer Keramik, wie etwa Aluminiumoxid oder aus einer Zirkondioxidkeramik (TZP oder ATZ), bestehen.

Aus der WO 2010/025386 A1 ist ein Osteosysnthesekörper in Form einer Schraube gemäß dem Oberbegriff von Anspruch 1 bekannt, die aus Metall, etwa aus Edelstahl, Titan, einer Titanlegierung, oder einer Kobalt-Chrom-Legierung besteht. Ferner ist die Schraube mit einem weicheren Material, wie etwa Polyetherketon oder Polyethylen beschichtet.

Vor diesem Hintergrund liegt der Erfindung die Aufgabe zu Grunde, einen Ostheosynthesekörper in Form einer Schraube und ein Verfahren zur Herstellung eines solchen anzugeben, womit eine besonders gute Verträglichkeit erreicht wird und allergische Reaktionen weitgehend vermieden werden.

Diese Aufgabe wird erfindungsgemäß durch einen Osteosynthesekörper aus einer Zirkondioxidkeramik gemäß Anspruch 1 gelöst.

Die Aufgabe der Erfindung wird auf diese Weise vollkommen gelöst.

Zusätzliche Weiterbildungen der Erfindung sind in den abhängigen Ansprüchen unter Schutz gestellt.

In der Medizin wird Zirkondioxid zwar bereits als Material für Gelenkköpfe bei künstlichen Hüftgelenken verwendet. Auch in der Zahnmedizin hat sich Zirkondioxid als Material für Wurzelstifte, als Gerüst für vollkeramische Kronen oder Brücken etabliert. Seit Anfang der 2000er Jahre wird Zirkondioxid auch als Material für vollkeramische Implantate genutzt (vgl. z.B. WO 03/045268 A1)

Einer Übertragung dieser Anwendungen auf eine Anwendung von Zirkondioxid für die Osteosynthese ist hierdurch jedoch nicht nahegelegt, da dem grundsätzlich die hohen Anforderungen entgegenstehen, die an die mechanische Stabilität und die Festigkeit der Osteosynthesekörper gestellt werden.

Es hat sich gezeigt, dass Osteosynthesekörper aus Zirkondioxidkeramik bioinert sind und gute osseointegrative Eigenschaften aufweisen. Ferner hat sich gezeigt, dass inzwischen mechanisch hochstabile Formteile und auch Schrauben aus reiner Zirkondioxidkeramik herstellbar sind, die eine hohe Biegefestigkeit und Bruchzähigkeit und einen geringen Elastizitätsmodul aufweisen und somit für die Osteosynthese geeignet sind. Insbesondere bei der Verwendung von TZP (tetragonalem, polykristallinem Zirkondioxid) lassen sich hochfeste Formkörper herstellen, die für die Osteosynthese geeignet sind.

Erfindungsgemäße Osteosynthesekörper in Form von Schrauben bestehen aus einer hochdichten Zirkondioxidkeramik aus tetragonalem, polykristallinem Zirkondioxid (TZP) oder aus einer Zirkondioxidkeramik-Mischkeramik mit Al₂O₃ (ATZ), mit einer Porosität von weniger als 0,1 %, bevorzugt von weniger als 0,01 %.

Diese zeichnen sich durch eine besonders hohe Festigkeit aus.

Grundsätzlich stehen bei der Anwendung von Osteosynthesekörpern aus Zirkondioxid zwei Alternativen zur Wahl. Die erste Alternative ist ein nur temporärer Einsatz bei der Fixation von Frakturen. Hierbei stehen die gute Verträglichkeit und Inertheit des Materials im Vordergrund. Das betreffende Osteosynthesematerial wird nach ausreichender Einheilzeit (meist mehrere Monate) wieder entfernt, wie dies auch bei Osteosynthesekörpern aus Stahl oder Titan der Fall ist. In einem solchen Fall wird eine glatte Oberfläche verwendet, d.h. eine Oberfläche, wie sie durch den Herstellprozess erzeugt wird (im Allgemeinen Sintern), ohne dass eine besondere Aufrauhung der Oberfläche erfolgt. Es kann zusätzlich eine Glättung z.B. durch eine finale Schleifbehandlung vorgesehen sein.

Soll der Osteosynthesekörper jedoch dauerhaft im Körper verbeiben, was durch die Inerheit des Materials ermöglicht wird, so kommt es zusätzlich auf eine besonders gute Osseointegration an.

Es hat sich gezeigt, dass eine hervorragende Osseointegration insbesondere durch eine mikroraue Oberfläche erreicht werden kann. Ein solche mikroraue Oberfläche lässt sich insbesondere erreichen, indem die Außenoberfläche mittels eines subtraktiven Verfahrens oberflächenbehandelt wird, insbesondere geätzt und/oder gestrahlt, insbesondere sandgestrahlt, wird. Hierbei stehen insbesondere milde Sandstrahlverfahren mit einer anschließenden Ätzbehandlung etwa mittels Flusssäure im Vordergrund.

Wenn die Osteosynthesekörper dauerhaft im Körper verbleiben, so können die guten Osseointegrationseigenschaften in Verbindung mit der osseointegrativen Kapazität von Zirkondioxid genutzt werden.

Insbesondere in Skelettbereichen, bei denen die mechanischen Belastungen nicht die hohen Belastungen des Gehapparates erreichen, also nicht im Bein- und Fußbereich,kann die etwas geringere mechanische Belastbarkeit von Zirkondioxid, insbesondere im Hinblick auf die Zugfestigkeit und Biegebruchfestigkeit im Vergleich zu metallischen Werkstoffen wie Titan oder Edelstahl, in Kauf genommen werden.

Insbesondere steht eine Verwendung von Osteosynthesekörpern aus Zirkondioxid im Kopfbereich, aber auch teilweise im Hand, Arm- oder Schulterbereich, im Vordergrund. Von besonderem Interesse ist hierbei eine Anwendung im Kopfbereich, etwa bei erheblichen Frakturen, Schussverletzungen, Kriegsverletzungen usw.

Die Aufgabe der Erfindung wird ferner durch ein Verfahren zum Herstellen eines Osteosynthesekörpers aus einer Zirkondioxidkeramik gemäß Anspruch 6 gelöst.

Etwa bei Verwendung von mit 3 Gew.-% Y₂O₃ teilstabilisiertem TZP lässt sich eine Biegefestigkeit von 1200 MPa erzielen.

Daneben ist es auch denkbar, eine Zirkondioxid-Mischkeramik zu verwenden, die mit Aluminiumoxid verstärkt ist (ATZ). Mit diesem Material lässt sich teilweise eine höhere Biegefestigkeit und ggf. auch eine verstärkte Bruchzähigkeit erzielen.

Es werden höchste Festigkeiten erzielt (Biegefestigkeit bis zu 1300 MPa bei TZP), wenn die Formgebung und Sinterung gleichzeitig durch heiß-isostatisches Pressen (HIP) erfolgt und anschließend eine mechanische Bearbeitung zur Erzeugung der gewünschten Form durchgeführt wird, was in der Regel durch Schleifen, Fräsen oder Bohren mit Diamantwerkzeugen erfolgt.

Da hierbei allerdings in der Regel Diamantwerkzeuge verwendet werden müssen und mittels HIP nur grobe Vorformen, etwa in Form von Platten oder Zylindern, durch HIP erzeugt werden können, ist dies ein sehr aufwändiges und kostenintensives Verfahren.

Alternativ kann daher die Formgebung durch übliche pulvertechnologische Verfahren, wie etwa Schlickergießen, Schleudergießen, uniaxiales Pressen, isostatisches Pressen oder elektrophoretische Abscheidung (EPD), erfolgen.

Vorzugsweise erfolgt anschließend zunächst ein Vorsintern zu einem Grünkörper, der anschließend im Grünzustand mechanisch bearbeitet wird, insbesondere gefräst, gesägt, geschliffen oder gebohrt wird, und der schließlich fertig gesintert wird.

Dies ermöglicht eine einfache Formgestaltung des Osteosynthesekörpers mittels mechanischer Bearbeitung im Grünzustand. Auf diese Weise ist auch eine individuelle Anpassung an einzelne Patienten ermöglicht.

Werden die Schrumpfungsmaße beim Sinterprozess ausreichend präzise zuvor berücksichtigt, was etwa durch eine CAD/CIM-Formgebung mittels automatisch gesteuerter Fräswerkzeuge erfolgen kann, so ist nach dem Sintern in der Regel gar keine oder nur eine minimale Nachbearbeitung notwendig.

Gemäß einer weiteren Variante der Erfindung ist es möglich, das Pulver mit einem organischen Binder zu einer druckfähigen Paste zu vermischen, mittels eines 3D-Druckers aufzutragen und anschließend zu sintern.

Beim Sintern verdampfen die organischen Bestandteile, und es entsteht ein hochdichter, porenarmer Körper. Durch den 3D-Druck lassen sich vielfältige Formen auf besonders einfache Weise erzeugen. Auf diese Weise ist auch eine patientenspezifische Anpassung möglich.

Gemäß einer weiteren Ausführung der Erfindung wird mikroskaliges Pulver mit einer mittleren spezifischen Oberfläche im Bereich von 5 bis 100 m²/g verwendet. Insbesondere eine größere spezifische Oberfläche im Bereich von 30, 50 m²/g oder mehr ist besonders bevorzugt, weil sich hierdurch besonders gute Sintereigenschaften ergeben und hochdichte, porenarme Körper erzeugt werden können. Gleichfalls ergeben sich niedrigere Sintertemperaturen.

Gemäß einer weiteren Ausführung der Erfindung wird nanoskaliges Pulver mit einer mittleren spezifischen Oberfläche von mehr als 100 m²/g, vorzugsweise von mehr als 200 m²/g verwendet. Nanoskalige TZP-Zirkondioxidpulver sind inzwischen kommerziell erhältlich. Zur Herstellung wird typischerweise die chemische Gasphasensynthese (CVS) eingesetzt.

Durch die Verwendung eines solchen Pulvers mit einer derart großen spezifischen Oberfläche lassen sich noch größere Festigkeiten erzielen, was für die Anwendung als Osteosynthesekörper besonders vorteilhaft ist.

Insbesondere ein Mischung aus mikroskaligem und nanoskaligem Pulver ist besonders vorteilhaft.

Die Sintertemperatur liegt vorzugsweise im Bereich von 850 °C bis 1800 °C, weiter bevorzugt im Bereich von 850 °C bis 1550 °C.

Die Sintertemperatur ist insbesondere von der Beschaffenheit des Pulvers und zwar insbesondere von der mittleren spezifischen Oberfläche, abhängig, d.h. je feiner das Pulver, desto niedriger liegt in der Regel die Sintertemperatur.

Bei Verwendung eines nanoskaligen Zirkondioxidpulvers erfolgt daher das Sintern bevorzugt im Bereich von 850 °C bis 1050 °C, vorzugsweise im Bereich von 900 °C bis 1000 °C.

Soweit ein Vorsintern zu einem Grünkörper durchgeführt wird, der ausreichend fest für eine mechanische Bearbeitung im Grünzustand ist, so erfolgt dies in der Regel bei einer Vorsintertemperatur, die 100 K bis 500 K unterhalb der Sintertemperatur liegt.

Es versteht sich, dass die vorstehend genannten und die nachfolgend noch zu erläuternden Merkmale nicht nur in der jeweils angegebenen Kombination, sondern auch in anderen Kombinationen oder in Alleinstellung verwendbar sind, ohne den Rahmen der Erfindung zu verlassen.

Weitere Merkmale und Vorteile der Erfindung ergeben sind aus der nachfolgenden Beschreibung bevorzugter Ausführungsbeispiele unter Bezugnahme auf die Zeichnung. Es zeigen:
Fig. 1 eine beispielhafte Ausführung einer Osteosyntheseplatte, die aus TZP besteht und
Fig. 2 eine perspektivische Darstellung einer erfindungsgemäßen Knochenschraube aus Zirkondioxid zur Osteosynthese, die mit einem selbstschneidenden Gewinde versehen ist.

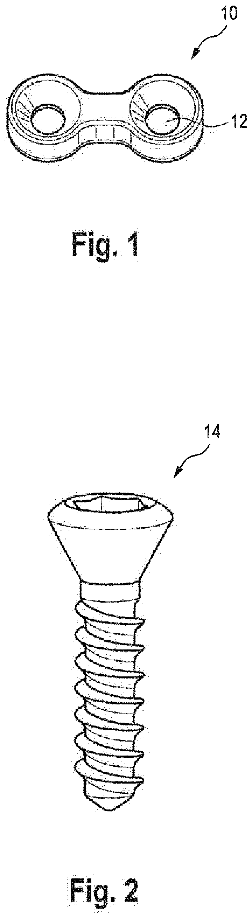

In Fig. 1 ist eine beispielhafte Osteosyntheseplatte 10 dargestellt, die als winkelstabile Platte ausgebildet ist, wie sie etwa zur Unterkieferfrakturbehandlung verwendet werden kann. Die Osteosynthesplatte 10 weist zwei konusförmig vertiefte Schraublöcher 12 zur Aufnahme von Ostheosyntheseschrauben 14 gemäß Fig. 2 auf.

Zur Herstellung der Osteosynthesplatte 10 wird zunächst ein kommerziell erhältliches TZP-Zirkondioxidpulver (mit 3 Gew.-% Y₂O₃ stabilisiert) mit einer mittleren spezifischen Oberfläche von etwa 50 m²/g isostatisch gepresst (Druck etwa 1000 bis 3000 bar, über 1 bis 100 Sekunden), dann bei etwa 900 °C vorgesintert, etwa 30 Minuten.

Der so erhaltene Grünkörper wird mittels einer automatisch gesteuerten Fräseinrichtung auf die gewünschte Endform bearbeitet, wobei das Schrumpfungsmaß für den späteren Sinterprozess berücksichtigt wird. Anschließend erfolgt ein Fertigsintern bei etwa 1350 °C über eine Zeit von etwa 30 bis 60 Minuten.

Eine besonders kostengünstige Herstellung ergibt sich, wenn der vorgesinterte Grünkörper durch Schlickergießen hergestellt wird.

Mittels eines automatisch gesteuerten Hochgeschwindigkeitsfräsers oder -Schleifers mit Diamantwerkzeug wird hieraus eine Osteosyntheseplatte 10 hergestellt.

Zur Herstellung von erfindungsgemäßen Osteosyntheseschrauben 14 wird zwecks höchster Festigkeit TZP-Pulver gemäß dem obigen Beispiel durch HIP gepresst (z.B. 1000 bis 3000 bar, 1200 bis 1300 °C, 2-30 Minuten). Die Schraube 14 wird hieraus durch Fräsen oder Schleifen mit Diamantwerkzeugen hergestellt.

Grundsätzlich ist es möglich, Osteosyntheseschrauben 14 gemäß Fig. 2 zu verwenden, die mit einem selbstschneidenden Gewinde versehen sind.

In Folge der dabei auftretenden höheren Flächenpressung sind teilweise nicht selbstschneidende Schrauben bevorzugt, die etwa mit einem Feingewinde versehen sind und leicht konisch zulaufen (nicht dargestellt). Dies stellt zwar höhere Anforderungen an den Operateur, da ein Gewindeschneiden erforderlich ist, bedeutet jedoch eine höhere Belastungssicherheit.

Ist das Osteosynthesesystem bestehend aus der Osteosyntheseplatte 10 und den zugehörigen Schrauben nur zur temporären Fixation vorgesehen, so muss die Oberfläche der Osteosyntheseplatte 10 nach dem Sintern nicht zusätzlich oberflächenbehandelt werden. Ggf. erfolgt eine maschinelle Glättung durch eine Schleif-Nachbehandlung.

Im Falle einer dauerhaften Implantation werden dagegen die Oberflächen der Osteosynthesekörper (also Platten 10 und Schrauben 14) einer milden Sandstrahlbehandlung gefolgt von einer Ätzbehandlung mit Flusssäure unterzogen, um eine besonders gute Osseointegration zu bewirken.

## Patentansprüche

1. Osteosynthesekörper aus einer Zirkondioxidkeramik, in Form einer Schraube, bestehend aus einer hochdichten Zirkondioxidkeramik mit einer Porosität von weniger als 0,1 % aus tetragonalem, polykristallinem Zirkondioxid (TZP) oder aus einer Zirkondioxidkeramik-Mischkeramik mit Al₂O₃ (ATZ).

2. Osteosynthesekörper nach Anspruch 1, in Form einer nicht selbstschneidenden, etwa mit einem Feingewinde versehenen, leicht konisch zulaufenden Schraube oder in Form einer selbstschneidenden Schraube.

3. Osteosynthesekörper nach Anspruch 1 oder 2, mit einer Porosität von weniger als 0,01 %.

4. Osteosynthesekörper nach einem der Ansprüche 1 bis 3, bei dem die Außenoberfläche aufgeraut ist.

5. Osteosynthesekörper nach Anspruch 4, bei dem die Außenoberfläche mittels eines subtraktiven Verfahrens oberflächenbehandelt ist, insbesondere geätzt und/oder gestrahlt, insbesondere sandgestrahlt, ist.

6. Verfahren zum Herstellen eines Osteosynthesekörpers gemäß einem der vorhergehenden Ansprüche, bei dem aus einem Pulver aus tetragonalem, polykristallinem Zirkondioxid (TZP) oder aus Zirkondioxidmischkeramikpulver (ATZ), ein Osteosnthesekörper in Form einer Schraube geformt und zu einer hochdichten Zirkondioxidkeramik mit einer Porosität von weniger als 0,1 % gesintert wird.

7. Verfahren nach Anspruch 6, bei dem das Pulver mittels HIP verdichtet und gesintert wird und anschließend mechanisch bearbeitet wird, insbesondere geschliffen wird.

8. Verfahren nach Anspruch 6, bei dem die Formgebung durch Schlickergießen, Schleudergießen, uniaxiales Pressen, isostatisches Pressen oder elektrophoretische Abscheidung (EPD) erfolgt.

9. Verfahren nach Anspruch 6, bei dem zunächst ein Formling aus Pulver und ggf. Zusätzen, wie etwa Binder, geformt wird, der zu einem Grünkörper vorgesintert wird, anschließend im Grünzustand mechanisch bearbeitet wird, insbesondere gefräst, gesägt, geschliffen oder gebohrt wird, und schließlich fertig gesintert wird.

10. Verfahren nach Anspruch 6, bei dem das Pulver mit einem Binder zu einer druckfähigen Paste vermischt wird, mittels eines 3D-Druckers aufgetragen wird und anschließend gesintert wird.

11. Verfahren nach einem der Ansprüche 6 bis 10, bei dem mikroskaliges Pulver mit einer mittleren spezifischen Oberfläche im Bereich von 5 bis 100 m²/g verwendet wird, oder bei dem nanoskaliges Pulver mit einer spezifischen Oberfläche von mehr als 100 m²/g, vorzugsweise von mehr als 200 m²/g verwendet wird, wobei vorzugsweise mikroskaliges und nanoskaliges Pulver gemischt werden.

12. Verfahren nach einem der Ansprüche 6 bis 11, bei dem das Sintern im Bereich von 850 °C bis 1750 °C, vorzugsweise im Bereich von 850 °C bis 1350 °C erfolgt, wobei vorzugsweise bei Verwendung eines nanoskaligen Zirkondioxidpulvers das Sintern im Bereich von 850 °C bis 1050 °C, vorzugsweise im Bereich von 900 °C bis 1000 °C, erfolgt.

13. Verfahren nach einem der Ansprüche 6 bis 12, bei dem ein Vorsintern zu einem Grünkörper bei einer Vorsintertemperatur erfolgt, die 100 K bis 500 K unterhalb der Sintertemperatur liegt.

14. Osteosynthesekörper nach einem der Ansprüche 1 bis 5 zur Verwendung in der dauerhaften Rekonstruktion von Knochenfrakturen oder Knochendefekten ohne nachfolgende Entfernung des Osteosynthesekörpers, insbesondere im Kopfbereich, im Bereich von Armen oder Schultern, oder zur Verwendung in der temporären Rekonstruktion von Knochenfrakturen oder Knochendefekten mit nachfolgender Entfernung des Osteosynthesekörpers, insbesondere im Kopfbereich, im Bereich von Händen, Armen oder Schultern.

15. Osteosynthesekörper zur Verwendung nach Anspruch 14, zur Verwendung in der Osteosynthese mit individuell anpassbaren Osteosynthesekörpern, die im vorgesinterten Grünzustand mechanisch bearbeitet und dann gesintert werden, oder mittels eines 3D-Druckers geformt und dann gesintert werden.

## Claims

1. An osteosynthesis body made of zirconium dioxide ceramic, in the form of a screw, consisting of a highly dense zirconium dioxide ceramic of tetragonal, polycrystalline zirconium dioxide (TZP) or of a zirconium dioxide mixture ceramic with Al₂O₃ (ATZ), having a porosity of less than 0.1 %.

2. The osteosynthesis body of claim 1, in the form of a non-self-tapping screw, such as provided with a fine thread, in the form of a slightly conically tapered screw, or in the form of a self-tapping screw.

3. The osteosynthesis body of claim 1 or 2, having a porosity of less than 0.5 %, preferably of less than 0.01 %.

4. The osteosynthesis body of any of claims 1 to 3, wherein the outer surface is roughened.

5. The osteosynthesis body of claim 4, wherein the outer surface is surface treated by means of a subtractive method, in particular is etched and/or blasted, in particular sand blasted.

6. A method for producing an osteosynthesis body according to any of the preceding claims, wherein from a powder of tetragonal, polycrystalline zirconium dioxide (TZP), or of a zirconium dioxide mixture ceramic powder (ATZ) an osteosynthesis body is formed to a screw, is densified and sintered to a porosity of less than 0.1 %.

7. The method of claim 6, wherein the powder is densified by means of HIP and sintered thereafter and is finally mechanically treated, in particular grinded.

8. The method of claim 6, wherein the shaping is performed by slip casting, centrifugal casting, uniaxial pressing, isostatic pressing, or electrophoretic deposition (EPD).

9. The method of claim 6, wherein initially a preform is formed from powder and optionally binders, is pre-sintered to a green body, is subsequently in the green state mechanically machined, in particular milled, sawn, ground or drilled, and is finally sintered to its final state.

10. The method of claim 6, wherein the powder is mixed with a binder to a printable paste, is applied by means of a 3D printer and is sintered thereafter.

11. The method of any of claims 6 to 10, wherein there is used a microscale powder having an average specific surface area in the range of 5 to 100 m g²/g, or a nanoscale powder having a specific surface area of more than 100 m²/g, preferably of more than 200 m²/g is used, wherein preferably microscale and nanoscale powders are mixed.

12. The method of any of claims 6 to 11, wherein the sintering is performed in the range of 850 °C to 1750 °C, preferably in the range of 850 °C to 1350 °C, wherein preferably when using a nanoscale zirconium dioxide powder sintering is performed in the range of 850 °C to 1050 °C, preferably in the range of 900 °C to 1000 °C.

13. The method of any of claims 6 to 12, wherein there is performed a pre-sintering to a green body at a pre-sintering temperature which is 100 K to 500 K below the sintering temperature.

14. An osteosynthesis body according to any of claims 1 to 5 for use in a permanent reconstruction of bone fractures or bone defects, without subsequent removal of the osteosynthesis body, in particular in the head region, in the region of arms or shoulders, or for use in the temporary reconstruction of bone fractures or bone defects with a subsequent removal of the osteosynthesis body, in particular in the head region, in the region of the hands, arms or shoulders.

15. An osteosynthesis body according to claim 14 for the osteosynthesis with individually customizable osteosynthesis bodies that in the pre-sintered green state are mechanically processed and sintered thereafter, or are shaped by means of a 3D printer and subsequently sintered.

## Revendications

1. Corps d'ostéosynthèse en céramique de dioxyde de zirconium, se présentant sous la forme d'une vis, comprenant une céramique de dioxyde de zirconium à haute densité dont la porosité est inférieure à 0,1 % qui comprend du dioxyde de zirconium polycristallin tétragonal (TZP), ou une céramique mixte de dioxyde de zirconium céramique comportant du Al₂O₃ (ATZ).

2. Corps d'ostéosynthèse selon la revendication 1, se présentant sous la forme d'une vis légèrement tronconique non auto-taraudeuse, munie par exemple d'un filetage fin, ou sous la forme d'une vis auto-taraudeuse.

3. Corps d'ostéosynthèse selon la revendication 1 ou 2, dont la porosité est inférieure à 0,01 %.

4. Corps d'ostéosynthèse selon l'une des revendications 1 à 3, dans lequel la surface extérieure est rendue rugueuse.

5. Corps d'ostéosynthèse selon la revendication 4, dans lequel la surface extérieure est traitée en surface, notamment gravée et/ou grenaillée, notamment sablée, au moyen d'un procédé soustractif.

6. Procédé de fabrication d'un corps d'ostéosynthèse selon l'une des revendications précédentes, dans lequel un corps d'ostéosynthèse est mis sous la forme d'une vis à partir d'une poudre de dioxyde de zirconium polycristallin tétragonal (TZP) ou de poudre de céramique mixte de dioxyde de zirconium (ATZ), et est fritté pour obtenir une céramique de dioxyde de zirconium à haute densité dont la porosité est inférieure à 0,1 %.

7. Procédé selon la revendication 6, dans lequel la poudre est compactée et frittée au moyen de HIP puis est traitée mécaniquement, notamment meulée.

8. Procédé selon la revendication 6, dans lequel la mise en forme est réalisée par coulée en barbotine, coulée centrifuge, pressage uniaxial, pressage isostatique ou dépôt électrophorétique (EPD).

9. Procédé selon la revendication 6, dans lequel un moulage est d'abord effectué à partir de poudre et éventuellement d'additifs, tel qu'un liant, qui est pré-fritté pour obtenir un corps cru, puis est traité mécaniquement à l'état cru, notamment fraisé, scié, meulé ou percé, et finalement soumis à un frittage final.

10. Procédé selon la revendication 6, dans lequel la poudre est mélangée à un liant pour former une pâte imprimable, est appliquée au moyen d'une imprimante 3D puis est frittée.

11. Procédé selon l'une des revendications 6 à 10, dans lequel de la poudre microscopique est utilisée dont la surface spécifique moyenne est de l'ordre de 5 à 100 m²/g, ou dans lequel de la poudre nanométrique est utilisée dont la surface spécifique est supérieure à 100 m²/g, de préférence supérieure à 200 m²/g, de préférence une poudre micrométrique et une poudre nanométrique étant mélangées.

12. Procédé selon l'une des revendications 6 à 11, dans lequel un frittage est effectué dans la gamme de 850 °C à 1750 °C, de préférence dans la gamme de 850 °C à 1350 °C, de préférence lors de l'utilisation d'une poudre de dioxyde de zirconium nanométrique le frittage étant effectué dans la gamme de 850 °C à 1050 °C, de préférence dans la gamme de 900 °C à 1000 °C.

13. Procédé selon l'une des revendications 6 à 12, dans lequel, pour obtenir un corps cru, un pré-frittage est effectué à une température de pré-frittage de 100 K à 500 K au-dessous de la température de frittage.

14. Corps d'ostéosynthèse selon l'une des revendications 1 à 5 destiné à être utilisé dans la reconstruction permanente de fractures osseuses ou de défauts osseux sans retrait ultérieur du corps d'ostéosynthèse, notamment au niveau de la tête, au niveau des bras ou des épaules, ou à être utilisé dans la reconstruction temporaire de fractures osseuses ou de défauts osseux avec retrait ultérieur du corps d'ostéosynthèse, en particulier au niveau de la tête, des mains, des bras ou des épaules.

15. Corps d'ostéosynthèse destiné à être utilisé selon la revendication 14, à être utilisé dans l'ostéosynthèse avec des corps d'ostéosynthèse adaptables individuellement qui sont traités mécaniquement à l'état cru pré-fritté puis frittés, ou mis en forme au moyen d'une imprimante 3D puis frittés.
